# EUROPÄISCHE PATENTSCHRIFT

(11) **EP 3 647 264 B1**
(45) Veröffentlichungstag und Bekanntmachung des Hinweises auf die Patenterteilung: **11.10.2023**
(21) Anmeldenummer: 18020567.6
(22) Anmeldetag: 31.10.2018
(51) Int. Cl.: C01B 3/52, C01B 3/48, C01B 3/12

(54) **VERFAHREN UND ANLAGE ZUM HERSTELLEN EINES GEREINIGTEN UND KONVERTIERTEN SYNTHESEGASES**
METHOD AND SYSTEM FOR THE PRODUCTION OF A PURIFIED AND CONVERTED SYNTHESIS GAS
PROCÉDÉ ET INSTALLATION DE PRODUCTION D'UN GAZ DE SYNTHÈSE ÉPURÉ ET CONVERTI

(43) Veröffentlichungstag der Anmeldung: 06.05.2020
(73) Patentinhaber: L'AIR LIQUIDE, SOCIÉTÉ ANONYME POUR L'ÉTUDE ET L'EXPLOITATION DES PROCÉDÉS GEORGES CLAUDE, 75007 Paris (FR)
(72) Erfinder: Walter, Stefan, 64347 Griesheim (DE)
(74) Vertreter: Dropsch, Holger

(56) Entgegenhaltungen:
- WO-A1-2009/065841
- US-A- 3 064 029

## Beschreibung

### Gebiet der Erfindung

Die Erfindung betrifft ein Verfahren zum Herstellen eines gereinigten und konvertierten Synthesegases, das neben den wesentlichen Synthesegasbestandteilen Wasserstoff (H₂) und Kohlenmonoxid (CO) auch acide Nebenbestandteile wie Kohlendioxid (CO₂) und Schwefelwasserstoff (H₂S) und ferner Spurenbestandteile wie beispielsweise Carbonylsulfid (COS), Ammoniak (NH₃), Cyanwasserstoff (HCN), Mercaptane (RSH) sowie Metallcarbonyle, insbesondere Eisen- und Nickelcarbonyle, umfasst. Dabei wird zunächst ein kohlenstoffhaltiger Einsatzstoff in einer Synthesegaserzeugungsstufe zu einem Rohsynthesegas umgesetzt, dass in einer nachfolgenden CO-Konvertierungszone hinsichtlich seines H₂/CO-Verhältnisses verändert und schließlich einer nach einem physikalischen Gaswaschverfahren mit Methanol als Absorptionsmittel arbeitenden Gaswaschzone zugeführt wird, in der der Gehalt des Synthesegases an unerwünschten Gasbestandteilen, insbesondere von aciden Gasbestandteilen, abgesenkt wird.

Die Erfindung betrifft auch eine Anlage zur Durchführung des erfindungsgemäßen Verfahrens.

### Stand der Technik

Aufgrund seiner großen Bedeutung als Einsatzstoff für zahlreiche chemische Synthesen, beispielsweise chemisch-industrieller Grundprodukte wie Methanol oder Ammoniak, ist die Herstellung von Synthesegas, also als wesentliche Bestandteile Wasserstoff (H₂) und Kohlenmonoxid (CO) umfassende Gasgemische, seit langem bekannt und vielfach im Schrifttum diskutiert. Eine Übersicht über den Stand der Technik bieten die Artikel unter den Stichworten "Gas Production" sowie "Ammonia - 4.5.1 Synthesis Gas Production", beide in Ullmann's Encyclopedia of Industrial Chemistry, Sixth Edition, 1998 Electronic Release, Verlag Wiley-VCH, sowie die Monographie "Gasification", 2nd Edition, C. Higman, M. van der Burgt, Gulf Professional Publishing (2008).

Als Edukte für die Synthesegaserzeugung dienen allgemein kohlenstoffhaltige Einsatzstoffe oder Einsatzstoffgemische, die in festem, flüssigem oder gasförmigem Aggregatzustand vorliegen können. Als Beispiele sind hier Kohle in stückiger Form oder Pulverform, Biomasse, Erdölfraktionen, Pyrolyseöle, Biogas oder Erdgas zu nennen. Bei der Umwandlung nicht-gasförmiger Einsatzstoffe in Synthesegas wird der Vorgang oft als Vergasung bezeichnet, wohingegen beim Einsatz von Erdgas eher von Reformierung, beispielsweise - je nach Verfahrensführung - von Dampfreformierung (Steamreforming) oder autothermer Reformierung (ATR) oder - bei Abwesenheit fester Katalysatoren - von partieller Oxidation (POX) gesprochen wird.

Als Produkt der Synthesegaserzeugung wird zunächst ein Rohsynthesegas erhalten, das in vielen Fällen hinsichtlich seines H₂/CO-Verhältnisses verändert und von bestimmten unerwünschten Nebenprodukten und Spurenbestandteilen befreit werden soll, um es als Einsatz für nachgeschaltete Verfahrens- oder Synthesestufen geeignet zu machen.

Die Einstellung des H₂/CO-Verhältnisses erfolgt über die CO-Konvertierungsreaktion, auch als Wassergas-Shift-Reaktion (WGS) oder CO-Shift-Reaktion bezeichnet, gemäß der Umsatzgleichung

CO + H₂O = CO₂ + H₂

Unter Zugabe von Wasserdampf reagiert demnach das CO zu CO₂ und H₂. Aufgrund der Reaktionsenthalpie von -41,2 kJ/mol verschiebt sich das chemische Gleichgewicht mit steigender Temperatur von den Reaktionsprodukten hin zu den Reaktionsedukten. Je nach angewandter Reaktionstemperatur wird dabei von Hochtemperatur (HTS)-, Mitteltemperatur (MTS)- oder Tieftemperatur (LTS)-Shift gesprochen.

Je nach Art der verwendeten Katalysatoren ist es ferner möglich, die Shift-Reaktion auch mit dem ungereinigten Rohsynthesegas durchzuführen. Dieses Verfahren wird als Rohgas-Shift oder auch - wegen der aciden Gasbestandteile, namentlich CO₂ und H₂S - als Sauergas-Shift bezeichnet. Sie kann auch mit Rohsynthesegasen durchgeführt werden, die signifikante Mengen an Schwefel, Ruß oder kondensierbaren Kohlenwasserstoffen enthalten. Eine typische Anwendung besteht in der CO-Konvertierung von Rohgasen aus der Schwerölvergasung, die nicht gekühlt und entschwefelt wurden, sondern nur heiß abgeschreckt ("gequencht") wurden, um den erforderlichen Dampf hinzuzufügen und Ruß zu entfernen. Eine weitere typische Anwendung besteht in der Konvertierung des Kohlenmonoxids in Rohgasen aus der Kohledruckvergasung, die nicht nur Schwefel, sondern auch gesättigte und ungesättigte Kohlenwasserstoffe, einschließlich Teere, enthalten. Zur Umwandlung beider Rohgasarten haben sich besonders Katalysatoren auf Basis von Cobalt/Molybdän bewährt. Diese sind schwefelresistent bzw. erreichen erst in Gegenwart sulfidischen Schwefels ihre volle Aktivität. So kann der Kohlenmonoxidgehalt (ca. 45 Vol.-%) eines Rohgases aus der partiellen Oxidation von Schweröl, das bei etwa 250 ºC Dampf/Trockengas-Verhältnis von ca. 0,8 in den CO-Shift-Reaktor eintritt, in einem zweistufigen CO-Shift-Reaktorsystem mit Cobalt/Molybdän-Katalysator auf ca. 1,6 Vol.-% abgesenkt werden. Die durch die Reaktionsenthalpie freigesetzte Wärme wird z. B. zur Erzeugung von Dampf mit hohem und niedrigem Druck und zur Vorwärmung von Speisewasser verwendet.

Verfahren zur Abtrennung von unerwünschten Begleitstoffen aus Rohsynthesegasen mittels physikalischer oder chemischer Absorption oder Gaswäsche sind aus dem Stand der Technik wohlbekannt. So können mit solchen Verfahren unerwünschte, acide Bestandteile aus durch Vergasung oder Reformierung von kohlenstoffhaltigen Einsatzstoffen erzeugten Rohsynthesegasen, beispielsweise Kohlendioxid (CO₂) und Schwefelwasserstoff (H₂S), aber auch weitere Bestandteile wie Carbonylsulfid (COS), Cyanwasserstoff (HCN) oder Mercaptane (RSH), von den erwünschten Synthesegasbestandteilen Wasserstoff (H₂) und Kohlenmonoxid (CO) sicher bis in den Spurenbereich entfernt werden. Ein bekanntes und häufig angewendetes Verfahren ist das Rectisol-Verfahren, das ebenfalls im oben genannten Schrifttum grundsätzlich beschrieben wird.

Im Rectisol-Verfahren erfolgt die Aufnahme der oben erwähnten, unerwünschten Störkomponenten durch kaltes, d. h. signifikant unter Umgebungstemperatur abgekühltes Methanol als Absorbens oder Waschmittel, wobei in einer Absorberkolonne, auch Waschkolonne genannt, ein intensiver Stoffaustausch zwischen dem Rohgas und dem Waschmittel bzw. Absorptionsmittel erfolgt. Dabei erhöht sich die Löslichkeit der unerwünschten Gasbestandteile drastisch mit sinkender Temperatur des Methanols und zunehmendem Druck, während sie für Wasserstoff und Kohlenmonoxid praktisch konstant bleibt. Methanol hat zudem den Vorteil, selbst bei Temperaturen bis hinab zu -75 °C noch eine geringe Viskosität und somit gute Stoff- und Wärmeübertragungseigenschaften aufzuweisen.

Das als Absorptionsmittel verwendete, mit den Störkomponenten beladene Methanol wird im Rectisol-Verfahren über Regenerierungsvorrichtungen im Kreis gefahren. In den Regenerierungsvorrichtungen wird das beladene Methanol von den absorbierten Gasen auf physikalischem Wege befreit. Dabei wird in einem ersten Regenerierungsschritt CO₂ durch Druckentspannung (sogenannte Flashregenerierung) und/oder Strippen mit einem Gas, beispielsweise Stickstoff, aus dem beladenen Methanol-Absorptionsmittel entfernt. In einem weiteren oder alternativen Regenerierungsschritt werden die schwefelhaltigen Gase, COS und H₂S, durch Erhitzen abgetrieben (sogenannte Heissregenerierung).

Beim Rectisol-Verfahren wird zwischen dem Standardverfahren und dem selektiven Rectisol-Verfahren unterschieden. Bei dem Standard-Rectisol-Verfahren werden die Begleitgase COS/H₂S und das CO₂ gemeinsam, in einem Absorptionsschritt aus dem Rohsynthesegas abgetrennt. Dagegen werden bei dem sogenannten selektiven Rectisol-Verfahren die schwefelhaltigen Begleitgase COS/H₂S und das CO₂ jeweils in separaten, nacheinander ablaufenden Absorptionsschritten aus dem Rohsynthesegas abgetrennt.

Nach Durchlaufen von zumeist mehreren Regenerierungsschritten werden mehrere Teilströme des von den Störkomponenten befreite Absorptionsmittel, beim Rectisol-Verfahren also klassischerweise das Methanol, zu der Absorberkolonne zurückgeführt. Das durch Heissregenerierung regenerierte Absorptionsmittel weist dabei die höchste Reinheit auf und wird daher zur Feinwäsche bzw. Feinabsorption von bereits vorgereinigtem Synthesegas verwendet; es stellt somit die abschließende Waschstufe dar, bevor das gereinigte Synthesegas als Reinsynthesegas die Absorberkolonne üblicherweise an deren oberem Ende verlässt.

Um ein Akkumulieren von Spurenkomponenten oder auch von Wasser im Methanol- Absorptionsmittel zu verhindern, ist es erforderlich, einen kleinen, mit den Spurenkomponenten oder anderen unerwünschten Bestandteilen beladenen Strom des Absorptionsmittels fortwährend aus dem Gaswaschverfahren auszuschleusen. So beschreibt die US-Patentpublikation US 3064029 A ein Absorptionsverfahren zur Reinigung von Synthesegas mit Methanol als Absorptionsmittel, wobei ein wasserbeladener Methanolstrom ausgeschleust und somit die Wasserkonzentration auf 2 mol-% begrenzt wird.

Dieser Ausschleusungsstrom wird dabei als Spülstrom, Purgestrom oder Bleedstrom bezeichnet. Er kann nachfolgend durch Verbrennen in einer geeigneten Verbrennungsanlage oder mittels eines Entsorgungsdienstleisters entsorgt werden. Nachteilig ist dabei allerdings, dass hierfür Kosten anfallen, insbesondere, falls eine geeignete Verbrennungsanlage in dem betrachteten Anlagenverbund gar nicht oder nicht in ausreichendem Maße zur Verfügung steht. Nachteilig ist zudem, dass der wird Spülstrom beim Verbrennen nur
thermisch, aber nicht stofflich genutzt wird. WO 2009/065841 A1 beschreibt ebenfalls das Ausleiten eines mit Spurenverunreinigungen beladenen, flüssigen Methanolspülstroms aus der Gaswaschzone eines Absorptionsverfahrens zur Reinigung von Synthesegas mit Methanol. Dabei wird der zurückgeführte Methanolspülstrom entweder zum Vergasungsreaktor oder zur Kühlsektion zurückgeführt. Demnach wird auch in dieser Ausgestaltung Methanol zerstörst.

### Beschreibung der Erfindung

Aufgabe der Erfindung ist es daher, ein Verfahren und eine Anlage anzugeben, die die genannten Nachteile des Stands der Technik vermeiden und die es insbesondere ermöglichen, ein gereinigtes und konvertiertes Synthesegas herzustellen, wobei der aus der Gaswäsche mit Methanol als Absorptionsmittel erhaltene Methanol-Spülstrom effizienter entsorgt oder genutzt wird.

Diese Aufgabe wird im Wesentlichen durch ein Verfahren mit den Merkmalen des Anspruchs 1 ff. und durch eine Anlage gemäß Anspruch 8 ff. gelöst. Weitere, insbesondere bevorzugte Ausgestaltungen des Verfahrens und der Anlage finden sich in den jeweiligen Unteransprüchen.

### Erfindungsgemäßes Verfahren:

Verfahren zum Herstellen eines gereinigten und konvertierten Synthesegases, enthaltend Wasserstoff (H₂) und Kohlenmonoxid (CO), umfassend folgende Verfahrensschritte:
a) Bereitstellen eines kohlenstoffhaltigen Vergasungs- oder Reformierungseinsatzstroms und Einleiten in eine Synthesegaserzeugungsstufe,
b) Umsetzen des kohlenstoffhaltigen Vergasungs- oder Reformierungseinsatzstroms in der Synthesegaserzeugungsstufe unter Synthesegaserzeugungsbedingungen zu einem Rohsynthesegasproduktstrom, der neben den Hauptbestandteilen H₂ und CO ferner Kohlendioxid (CO₂) und Schwefelwasserstoff (H₂S) als acide Synthesegasbestandteile sowie Neben- und Spurenkomponenten wie Carbonylsulfid (COS), Ammoniak (NH₃), Cyanwasserstoff (HCN), Mercaptane (RSH), Metallcarbonyle enthält, Ausleiten des Rohsynthesegasproduktstroms aus der Synthesegaserzeugungsstufe,
c) Einleiten des Rohsynthesegasproduktstroms in eine CO-Konvertierungszone (CO-Shift-Zone), Umsetzen des Rohsynthesegasproduktstroms in der CO-Konvertierungszone unter CO-Konvertierungsbedingungen in einen an H₂ angereicherten und an CO abgereicherten CO-Konvertierungsproduktstrom, Ausleiten des CO-Konvertierungsproduktstroms aus der CO-Konvertierungszone,
d) Einleiten des CO-Konvertierungsproduktstroms in eine nach einem physikalischen Gaswaschverfahren mit Methanol als Absorptionsmittel arbeitenden Gaswaschzone, in der das Methanol im Kreis geführt und kontinuierlich regeneriert wird, Ausleiten eines an aciden Gasbestandteilen wie CO₂ und H₂S abgereicherten Reinsynthesegasproduktstroms aus der Gaswaschzone, Ausleiten eines oder mehrerer an aciden Gasbestandteilen wie CO₂ und H₂S angereicherter Stoffströme aus der Gaswaschzone, Ausleiten eines mit Spurenverunreinigungen beladenen, flüssigen Methanolspülstroms aus der Gaswaschzone,
wobei der Methanolspülstrom mindestens teilweise in die CO-Konvertierungszone zurückgeführt wird und dem Rohsynthesegasproduktstrom vor Einleiten in die CO-Konvertierungszone zugegeben wird.

### Erfindungsgemäße Anlage:

Anlage zum Herstellen eines gereinigten und konvertierten Synthesegases, enthaltend Wasserstoff (H₂) und Kohlenmonoxid (CO), umfassend folgende, miteinander in Fluidverbindung stehende Bestandteile, Bauelemente und Funktionsgruppen:
a) Eine Synthesegaserzeugungsstufe, Mittel zum Bereitstellen eines kohlenstoffhaltigen Vergasungs- oder Reformierungseinsatzstroms und Mittel zum Einleiten desselben in die Synthesegaserzeugungsstufe,
b) Mittel zum Umsetzen des kohlenstoffhaltigen Vergasungs- oder Reformierungseinsatzstroms in der Synthesegaserzeugungsstufe unter Synthesegaserzeugungsbedingungen zu einem Rohsynthesegasproduktstrom, der neben den Hauptbestandteilen H₂ und CO ferner Kohlendioxid (CO₂) und Schwefelwasserstoff (H₂S) als acide Synthesegasbestandteile sowie Neben- und Spurenkomponenten wie Carbonylsulfid (COS), Ammoniak (NH₃), Cyanwasserstoff (HCN), Mercaptane (RSH), Metallcarbonyle enthält, Mittel zum Ausleiten des Rohsynthesegasproduktstroms aus der Synthesegaserzeugungsstufe,
c) eine CO-Konvertierungszone (CO-Shift-Zone), Mittel zum Einleiten des Rohsynthesegasproduktstroms in die CO-Konvertierungszone, Mittel zum Umsetzen des Rohsynthesegasproduktstroms in der CO-Konvertierungszone unter CO-Konvertierungsbedingungen in einen an H₂ angereicherten und an CO abgereicherten CO-Konvertierungsproduktstrom, Mittel zum Ausleiten des CO-Konvertierungsproduktstroms aus der CO-Konvertierungszone,
d) eine nach einem physikalischen Gaswaschverfahren mit Methanol als Absorptionsmittel arbeitende Gaswaschzone, in der das Methanol im Kreis geführt und kontinuierlich regeneriert wird, Mittel zum Einleiten des CO-Konvertierungsproduktstroms in die Gaswaschzone, Mittel zum Ausleiten eines an aciden Gasbestandteilen wie CO₂ und H₂S abgereicherten Reinsynthesegasproduktstroms aus der Gaswaschzone, Mittel zum Ausleiten eines oder mehrerer an aciden Gasbestandteilen wie CO₂ und H₂S angereicherter Stoffströme aus der Gaswaschzone, Mittel zum Ausleiten eines mit Spurenverunreinigungen beladenen, flüssigen Methanolspülstroms aus der Gaswaschzone,
wobei ferner Mittel umfasst werden, die es gestatten, den Methanolspülstrom mindestens teilweise in die CO-Konvertierungszone zurückzuführen und
dem Rohsynthesegasproduktstrom vor Einleiten in die CO-Konvertierungszone zuzugeben.

Dabei stellt die mit Buchstaben oder Zahlen nummerierte Merkmalsgliederung in den Ansprüchen nicht zwingenderweise die zeitliche Abfolge der Verfahrensschritte oder die räumliche Anordnung von Anlagenbestanteilen dar.

Unter Fluidverbindung zwischen zwei Bereichen wird jegliche Art von Verbindung verstanden, die es ermöglicht, dass ein Fluid, beispielsweise das flüssige Absorptionsmittel, von dem einen zu dem anderen der beiden Bereiche strömen kann, unbeachtlich etwaiger zwischengeschalteter Bereiche, Bauteile, Armaturen oder Vorrichtungen.

Als Vergasungs- oder Reformierungseinsatzstrom wird ein Stoffstrom bezeichnet, der Kohlenstoffträger umfasst, die unter Vergasungs- oder Reformierungsbedingungen zu Synthesegasbestandteilen umgesetzt werden können.

Als Kohlenstoffträger werden dabei alle Stoffe oder Stoffgemische verstanden, die Kohlenstoff in unter Synthesegaserzeugungsbedingungen zu Synthesegasbestandteilen umsetzbarer Form enthalten. Als Beispiele können hier Erdgas, Steinkohle, Braunkohle, Biomasse sowie kohlenstoffhaltige Abfälle oder Nebenprodukte, z. B. Raffinerierückstände oder Pyrolyseöle, genannt werden.

Mit dem Begriff Biomasse wird die Stoffmasse von Lebewesen oder deren Teile bzw. Körperteile bezeichnet. Im weiteren Sinne wird darunter auch fossile Biomasse wie beispielsweise Kohle, Erdöl oder Erdgas verstanden.

Unter einer Synthesegaserzeugungsstufe werden alle Vorrichtungen bzw. Verfahrensabschnitte verstanden, die dazu geeignet sind, aus einem Kohlenstoffträger enthaltenden Einsatzstrom ein Wasserstoff und Kohlenmonoxid enthaltendes Gasgemisch, also Synthesegas, zu erzeugen. Bei der Umwandlung nicht-gasförmiger Kohlenstoffträger zu Synthesegas wird der Vorgang als Vergasung bezeichnet, wohingegen beim Einsatz von Erdgas eher von Reformierung, beispielsweise - je nach Verfahrensführung - von Dampfreformierung (Steamreforming) oder autothermer Reformierung (ATR) oder - bei Abwesenheit fester Katalysatoren - von partieller Oxidation (POX) gesprochen wird.

Unter CO-Konvertierungszone wird ein räumlich abgegrenzter Bereich verstanden, der so beschaffen ist, dass in seinem Inneren die CO-Konvertierungsreaktion durchgeführt werden kann. Dazu umfasst die CO-Konvertierungszone Mittel zum Einleiten eines zu konvertierenden Synthesegasstroms und von Wasser als Reaktionspartner, Mittel zum Ausleiten eines konvertierten, d. h. eines an Wasserstoff angereicherten und an CO abgereicherten Synthesegasstroms und einen für die CO-Konvertierungsreaktion aktiven Katalysator. Die CO-Konvertierungszone kann beispielsweise einen CO-Konvertierungsreaktor, insbesondere auch mehrere CO-Konvertierungsreaktoren umfassen, die jeweils ein einziges, insbesondere aber auch mehrere Katalysatorbetten enthalten. Die CO-Konvertierungsreaktoren und/oder die Katalysatorbetten können dabei nacheinander und/oder parallel von dem zu konvertierenden Synthesegasstrom durchströmt werden.

Unter Synthesegaserzeugungsbedingungen bzw. CO-Konvertierungsbedingungen werden physikalisch-chemische Bedingungen verstanden, die eine mindestens teilweise, bevorzugt eine technisch relevante, beispielsweise eine weitgehend vollständige Umsetzung der in dem kohlenstoffhaltigen Vergasungs- oder Reformierungseinsatzstrom enthaltenen Kohlenstoffträger mit Vergasungsmitteln wie Sauerstoff, Luft und/oder Wasserdampf zu Synthesegasbestandteilen bzw. des im Rohsynthesegas enthaltenen Kohlenmonoxids in Kohlendioxid und Wasserstoff gestatten. Sie sind in beiden Fällen aus dem Stand der Technik an sich bekannt und umfassen neben dem Zuführen des bzw. der Vergasungsmittel auch die Einstellung geeigneter Temperaturen. Die genauen Synthesegaserzeugungsbedingungen bzw. CO-Konvertierungsbedingungen wird der Fachmann in Abhängigkeit von dem umzusetzenden Kohlenstoffträger bzw. dem gewünschten Konvertierungsgrad geeignet auswählen. Insbesondere im Falle der CO-Konvertierung wird er dabei auch den Einfluss der physikalisch-chemischen Bedingungen auf die Lage des Reaktionsgleichgewichts im Zusammenwirken mit der Reaktionskinetik berücksichtigen.

Unter einem Mittel wird eine Sache verstanden, die die Erreichung eines Zieles ermöglicht oder dabei behilflich ist. Insbesondere werden unter Mitteln zum Durchführen eines bestimmten Verfahrensschrittes alle diejenigen physischen Gegenstände verstanden, die der Fachmann in Betracht ziehen würde, um diesen Verfahrensschritt durchführen zu können. Beispielsweise wird der Fachmann als Mittel zum Einleiten oder Ausleiten eines Stoffstroms alle Transport- und Fördervorrichtungen, also z. B. Rohrleitungen, Pumpen, Verdichter, Ventile, in Betracht ziehen, die ihm aufgrund seines Fachwissens zur Durchführung dieses Verfahrensschrittes notwendig oder sinnvoll erscheinen.

Druckangaben in der Einheit bar(a) beziehen sich auf den Absolutdruck in bar, absolut. Druckangaben in der Einheit bar(g) beziehen sich auf den Überdruck in bar.

Der Erfindung liegt die Erkenntnis zugrunde, dass der CO-Konvertierungskatalysator auch aktiv ist für einen effektiven Abbau des Methanols, d. h. die Aufspaltung des Methanols in die Synthesegasbestandteile CO und H₂ in Umkehrung der Methanol-Synthesegleichung:

CO + 2 H₂ = CH₃OH

In vorteilhafter Weise kann somit das aus dem Gaswaschverfahren ausgeschleuste Methanol in den Synthesegaspool zurückgeführt und somit stofflich genutzt werden. Überraschenderweise erfolgt dabei zusätzlich ein Abbau unerwünschter Störkomponenten bzw. Spurenverunreinigungen, beispielsweise von Cyanwasserstoff.

### Bevorzugte Ausgestaltungen der Erfindung

Eine besondere Ausgestaltung des erfindungsgemäßen Verfahrens ist dadurch gekennzeichnet, dass der Rohsynthesegasproduktstrom vor dem Einleiten in die CO-Konvertierungszone ein Schutzbett durchläuft, das mit einem für Metallcarbonyle selektiven Adsorbens oder Absorbens gefüllt ist, wobei der Methanolspülstrom stromaufwärts des Schutzbettes dem Rohsynthesegasproduktstrom zugegeben wird. Auf diese Weise wird verhindert, dass die Metallcarbonyle in die CO-Konvertierungszone gelangen und dann den dort angeordneten CO-Konvertierungskatalysator vergiften.

Wenn der Methanolspülstrom stromaufwärts des Schutzbettes dem Rohsynthesegasproduktstrom zugegeben wird, sieht eine besondere Ausgestaltung vor, dass der Methanolspülstrom dem Rohsynthesegasproduktstrom in flüssiger Form zugegeben wird. Vorteilhaft ist es dabei, dass das Schutzbett gleichsam als statischer Mischer genutzt wird, so dass eine homogene Verteilung des Methanolspülstroms in dem Rohsynthesegasproduktstrom erfolgen kann.

Wenn der Methanolspülstrom stromaufwärts des Schutzbettes dem Rohsynthesegasproduktstrom zugegeben wird, sieht eine alternative Ausgestaltung vor, dass der Methanolspülstrom vor oder während seiner Zugabe zu dem Rohsynthesegasproduktstrom verdampft wird, wobei die Verdampfungswärme durch direkten oder indirekten Wärmetausch mit dem Rohsynthesegasproduktstrom zugeführt wird. Der Methanolspülstrom kann demnach direkt in flüssiger Form, beispielsweise durch Eindüsen oder Versprühen, dem Rohsynthesegasproduktstrom zugegeben werden und wird durch direkten Wärmetausch mit dem Rohsynthesegasproduktstrom verdampft. Alternativ kann der Methanolspülstrom auch vor Zusammenführen mit dem Rohsynthesegasproduktstrom verdampft werden, wobei die Verdampfungswärme beispielsweise mittels indirektem Wärmetausch mit dem Rohsynthesegasproduktstrom in einem geeigneten Wärmetauscher zugeführt werden kann. In beiden Fällen wirkt das durchströmte Schutzbett homogenisierend für das erhaltene Gasgemisch.

Eine weitere Ausgestaltung des erfindungsgemäßen Verfahrens ist dadurch gekennzeichnet, dass die CO-Konvertierungszone nach dem Prinzip der Rohgaskonvertierung (Rohgasshift) arbeitet. Die hierbei verwendeten Katalysatoren sind aktiv für die Umwandlung von Methanol in Synthesegasbestandteile und unempfindlich gegenüber etwaige Spurenkomponenten und Verunreinigungen in dem Methanolspülstrom.

Eine weitere, besondere Ausgestaltung des erfindungsgemäßen Verfahrens ist dadurch gekennzeichnet, dass die CO-Konvertierungszone mehrere Bereiche umfasst, die mit einem oder mehreren für die Rohgaskonvertierung aktiven Katalysatoren befüllt sind. Die mehrstufige Durchführung der CO-Konvertierung hat sich in der Technik bewährt, da auf diese Weise mehr Freiheitsgrade zur Einstellung der jeweils optimalen Reaktionsbedingungen in den einzelnen Katalysatorbetten vorhanden sind, beispielsweise durch Zwischenkühlung oder Feedeindosierung zwischen den einzelnen Katalysatorbetten. Vorteilhaft ist ferner, dass im Falle von in dem Methanolspülstrom enthaltenen Verunreinigungen, die als Katalysatorgifte wirken, vor allem das erste Katalysatorbett in Strömungsrichtung betroffen ist, so dass gegebenenfalls nur dieses regeneriert oder ausgetauscht werden muss, nicht aber das gesamte Katalysatorinventar der CO-Konvertierungszone.

In einer weiteren, besonderen Ausgestaltung des erfindungsgemäßen Verfahrens ist vorgesehen, dass die Gaswaschzone eine Heissregenerierungsvorrichtung zur Regenerierung von mit aciden Gasbestandteilen beladenem Methanol umfasst, wobei der mit Spurenverunreinigungen beladene, flüssigen Methanolspülstrom aus dem Sumpfprodukt und/oder dem Rücklauf der Heissregenerierungsvorrichtung gewonnen und aus der Heissregenerierungsvorrichtung ausgeleitet wird. Da sich die Spurenverunreinigungen im Sumpf der Heissregenerierungsvorrichtung besonders stark anreichern, stellt dies einen besonders geeigneten Ort zur Entnahme des Methanolspülstroms dar.

Eine besondere Ausgestaltung der erfindungsgemäßen Anlage ist dadurch gekennzeichnet, dass ferner ein Schutzbett umfasst wird, das mit einem für Metallcarbonyle selektiven Adsorbens oder Absorbens gefüllt ist und das stromaufwärts der CO-Konvertierungszone angeordnet ist, so dass der Rohsynthesegasproduktstrom vor dem Einleiten in die CO-Konvertierungszone das Schutzbett durchläuft, und Mittel, die es gestatten, dass der Methanolspülstrom stromaufwärts des Schutzbettes dem Rohsynthesegasproduktstrom zugegeben wird. Auf diese Weise wird verhindert, dass die Metallcarbonyle in die CO-Konvertierungszone gelangen und dann den dort angeordneten CO-Konvertierungskatalysator vergiften.

Wenn der Methanolspülstrom stromaufwärts des Schutzbettes dem Rohsynthesegasproduktstrom zugegeben wird, sieht eine besondere Ausgestaltung der erfindungsgemäßen Anlage vor, dass Mittel umfasst werden, die es gestatten, dass der Methanolspülstrom dem Rohsynthesegasproduktstrom in flüssiger Form zugegeben wird. Vorteilhaft ist es dabei, dass das Schutzbett gleichsam als statischer Mischer genutzt wird, so dass eine homogene Verteilung des Methanolspülstroms in dem Rohsynthesegasproduktstrom erfolgen kann.

Wenn der Methanolspülstrom stromaufwärts des Schutzbettes dem Rohsynthesegasproduktstrom zugegeben wird, sieht eine alternative Ausgestaltung der erfindungsgemäßen Anlage vor, dass Mittel umfasst werden, die es gestatten, dass der Methanolspülstrom vor oder während seiner Zugabe zu dem Rohsynthesegasproduktstrom verdampft wird, wobei die Verdampfungswärme durch direkten oder indirekten Wärmetausch mit dem Rohsynthesegasproduktstrom zugeführt wird. Der Methanolspülstrom kann demnach direkt in flüssiger Form, beispielsweise durch Eindüsen oder Versprühen, dem Rohsynthesegasproduktstrom zugegeben werden und wird durch direkten Wärmetausch mit dem Rohsynthesegasproduktstrom verdampft. Alternativ kann der Methanolspülstrom auch vor Zusammenführen mit dem Rohsynthesegasproduktstrom verdampft werden, wobei die Verdampfungswärme beispielsweise mittels indirektem Wärmetausch mit dem Rohsynthesegasproduktstrom in einem geeigneten Wärmetauscher zugeführt werden kann. In beiden Fällen wirkt das durchströmte Schutzbett homogenisierend für das erhaltene Gasgemisch.

Eine weitere Ausgestaltung der erfindungsgemäßen Anlage ist dadurch gekennzeichnet, dass die CO-Konvertierungszone nach dem Prinzip der Rohgaskonvertierung (Rohgasshift) arbeitet. Die hierbei verwendeten Katalysatoren sind aktiv für die Umwandlung von Methanol in Synthesegasbestandteile und unempfindlich gegenüber etwaige Spurenkomponenten und Verunreinigungen in dem Methanolspülstrom.

Eine weitere, besondere Ausgestaltung der erfindungsgemäßen Anlage ist dadurch gekennzeichnet, dass die CO-Konvertierungszone mehrere Bereiche umfasst, die mit einem oder mehreren für die Rohgaskonvertierung aktiven Katalysatoren befüllt sind. Die mehrstufige Durchführung der CO-Konvertierung hat sich in der Technik bewährt, da auf diese Weise mehr Freiheitsgrade zur Einstellung der jeweils optimalen Reaktionsbedingungen in den einzelnen Katalysatorbetten vorhanden sind, beispielsweise durch Zwischenkühlung oder Feedeindosierung zwischen den einzelnen Katalysatorbetten. Vorteilhaft ist ferner, dass im Falle von in dem Methanolspülstrom enthaltenen Verunreinigungen, die als Katalysatorgifte wirken, vor allem das erste Katalysatorbett in Strömungsrichtung betroffen ist, so dass gegebenenfalls nur dieses regeneriert oder ausgetauscht werden muss, nicht aber das gesamte Katalysatorinventar der CO-Konvertierungszone.

In einer weiteren, besonderen Ausgestaltung der erfindungsgemäßen Anlage ist vorgesehen, dass die Gaswaschzone eine Heissregenerierungsvorrichtung zur Regenerierung von mit aciden Gasbestandteilen beladenem Methanol umfasst, wobei der mit Spurenverunreinigungen beladene, flüssigen Methanolspülstrom aus dem Sumpfprodukt und/oder dem Rücklauf der Heissregenerierungsvorrichtung gewonnen und aus der Heissregenerierungsvorrichtung ausgeleitet wird. Da sich die Spurenverunreinigungen im Sumpf der Heissregenerierungsvorrichtung besonders stark anreichern, stellt dies einen besonders geeigneten Ort zur Entnahme des Methanolspülstroms dar.

### Ausführungs- und Zahlenbeispiele

Weitere Merkmale, Vorteile und Anwendungsmöglichkeiten der Erfindung ergeben sich auch aus der nachfolgenden Beschreibung eines Ausführungs- und Zahlenbeispiels und der Zeichnungen. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbeziehung.

Es zeigen
- Fig. 1: eine schematische Darstellung eines Verfahrens bzw. einer Anlage gemäß Stand der Technik,
- Fig. 2: eine schematische Darstellung einer beispielhaften Ausgestaltung eines Verfahrens bzw. einer Anlage gemäß der Erfindung.

In beiden Figuren wird die graphische Darstellung auf jeweils den Anlagenteil beschränkt, der die CO-Konvertierung (CO-Shift) betrifft.

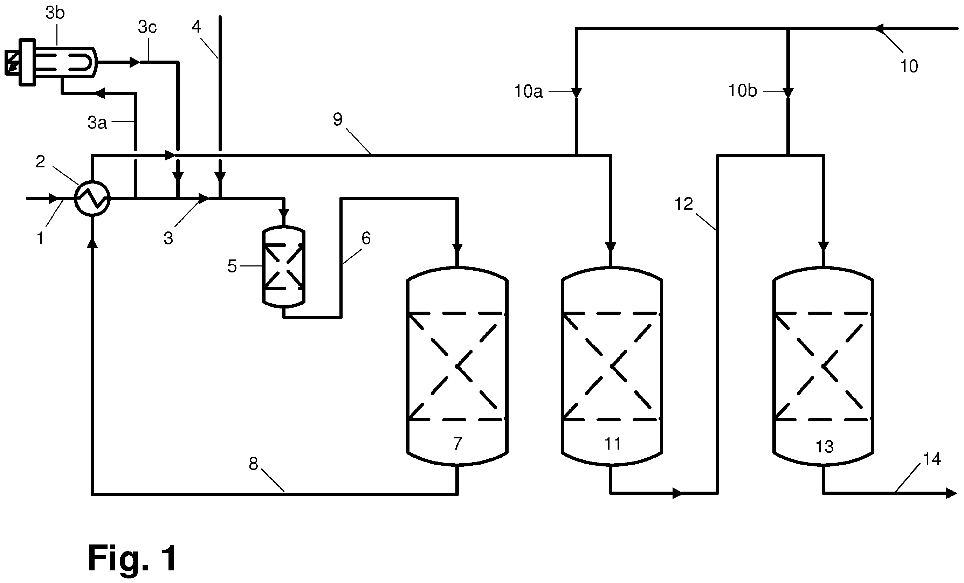

In der in Fig. 1 schematisch dargestellten Ausgestaltung eines Verfahrens bzw. einer Anlage gemäß des Stands der Technik wird über Leitung 1 Rohsynthesegas aus einer Anlage zur Kohlevergasung (nicht gezeigt) herangeführt und in Wärmetauscher 2 im indirekten Wärmetausch gegen heißes Produktgas des ersten Reaktors der mehrstufig ausgestalteten CO-Konvertierungszone (CO-Shift-Zone) aufgeheizt. Das Rohsynthesegas enthält dabei neben den erwünschten Synthesegasbestandteilen Wasserstoff und Kohlenmonoxid unter anderem auch die unerwünschten, aciden Synthesegasbestandteile Kohlendioxid und Schwefelwasserstoff sowie weitere organische und anorganische Schwefelverbindungen wie z. B. Carbonylsulfid (COS), Ammoniak (NH₃), Cyanwasserstoff (HCN), Mercaptane (RSH), Metallcarbonyle. Über Leitung 3 wird das aufgeheizte Rohsynthesegas aus dem Wärmetauscher 2 ausgeleitet.

Da bei der Inbetriebnahme des Verfahrens bzw. der Anlage zunächst noch kein heißes CO-Konvertierungs-Produktgas zur Verfügung steht, wird das von der Kohlevergasung herangeführte Rohsynthesegas über Leitung 3a zum Inbetriebnahmeheizer 3b geführt, dort erwärmt und über Leitung 3c zu Leitung 3 zurückgeführt.

Über Leitung 4 wird dem über Leitung 3 herangeführten, aufgeheizten Rohsynthesegas Wasser in Form von Hochdruckdampf als Reaktionspartner für die CO-Konvertierung zugeführt. Das somit erhaltene Reaktionsgemisch wird sodann über Leitung 3 einem in einem Behälter 5 enthaltenen Schutzbett zugeführt, das mit einem für Metallcarbonyle selektiven Adsorbens oder Absorbens gefüllt ist. Auf diese Weise werden die Metallcarbonyle aus dem Reaktionsgemisch entfernt, so dass es nicht mehr zu einer Vergiftung der nachgeschalteten Katalysatorstufen kommen kann.

Über Leitung 6 wird das von Carbonylen befreite Reaktionsgemisch aus dem Behälter 5 ausgeleitet und dem ersten Reaktor 7 einer mehrstufig, hier dreistufig ausgestalteten CO-Konvertierungszone 7, 11, 13 zugeführt. Im vorliegenden Beispiel handelt es sich um eine Rohgaskonvertierung bzw. Sauergas-Shift. Jede Reaktionsstufe besteht aus einem Festbettreaktor, der mit einer Schüttung eines festen, körnigen, für die Sauergas-Shift aktiven Katalysator befüllt ist. Der kommerziell erhältliche Katalysator basiert auf den Metallen Cobalt und Molybdän als Aktivkomponenten. Die anzuwendenden Reaktionsbedingungen sind daher durch den Lieferanten vorgegeben und dem Fachmann bekannt.

Da die CO-Konvertierungsreaktion in Reaktor 7 exotherm verläuft, tritt das CO-Konvertierungsproduktgas der ersten Reaktionsstufe mit einer Temperatur aus Reaktor 7 aus, die über der Eintrittstemperatur liegt. Daher wird das Produktgas mittels Leitung 8 aus Reaktor 7 ausgeleitet und der Mantelseite des Wärmetauschers 2 zugeführt, wo es im indirekten Wärmetausch das über Leitung 1 herangeführte Rohsynthesegas aufheizt und dabei selber abgekühlt wird. Das somit abgekühlte CO-Konvertierungsproduktgas der ersten Reaktionsstufe wird über Leitung 9 aus dem Wärmetauscher 2 ausgeleitet, mit verdampftem, über Leitungen 10 und 10a herangeführten Kesselspeisewasser vermischt und sodann dem zweiten Reaktor 11 der dreistufig ausgestalteten CO-Konvertierungszone 7, 11, 13 zugeführt. Für den Reaktortyp des Reaktors 11 und die Art des verwendeten Katalysators gilt das oben Gesagte.

In Reaktor 11 wird das CO-Konvertierungsproduktgas der ersten Reaktionsstufe weiter zu den CO-Konvertierungsprodukten H₂ und CO₂ umgesetzt, wobei der Umsatz kleiner ist als derjenige in Reaktor 7 und die Reaktion somit auch weniger stark exotherm verläuft. Daher wird das Konvertierungsproduktgas der zweiten Reaktionsstufe über Leitung 12 aus Reaktor 11 ausgeleitet und ohne weiteren Wärmetausch der dritten Reaktionsstufe zugeführt, die durch Reaktor 13 gebildet wird. Vor dem Einleiten in Reaktor 13 wird dem Konvertierungsproduktgas der zweiten Reaktionsstufe über Leitungen 10 und 10b zuvor verdampftes Kesselspeisewasser zugeführt und mit diesem vermischt.

Reaktor 13 bildet die abschließende Reaktionszone der dreistufig ausgestalteten CO-Konvertierungszone 7, 11, 13. Für den Reaktortyp des Reaktors 13 und die Art des verwendeten Katalysators gilt das oben Gesagte.

Über Leitung 14 wird das gasförmige CO-Konvertierungsendprodukt aus dem Reaktor ausgeleitet und einer in der Figur nicht gezeigten, nach einem physikalischen Gaswaschverfahren mit Methanol als Absorptionsmittel arbeitenden Gaswaschzone zugeführt. In dieser wird das Methanol im Kreis geführt und kontinuierlich in mehreren Regenerierungsstufen regeneriert, die als Flashregenerierungs- oder Heissregenerierungsvorrichtungen ausgestaltet sind.

Aus der Gaswaschzone werden ein an aciden Gasbestandteilen wie CO₂ und H₂S abgereicherten Reinsynthesegasproduktstrom, ein oder mehrere an aciden Gasbestandteilen wie CO₂ und H₂S angereicherter Stoffströme und ein mit Spurenverunreinigungen beladener, flüssiger Methanolspülstrom ausgeleitet. Der Methanolspülstrom wird dabei in vorteilhafter Weise aus dem Sumpfprodukt und/oder dem Rücklauf der Heissregenerierungsvorrichtung gewonnen und aus der Heissregenerierungsvorrichtung ausgeleitet, da sich insbesondere dort die mit dem Spülstrom zu entfernenden Verunreinigungen anreichern. Dieser Methanolspülstrom wird gemäß Stand der Technik entweder durch Verbrennen thermisch entsorgt oder einer Entsorgungsfirma übergeben.

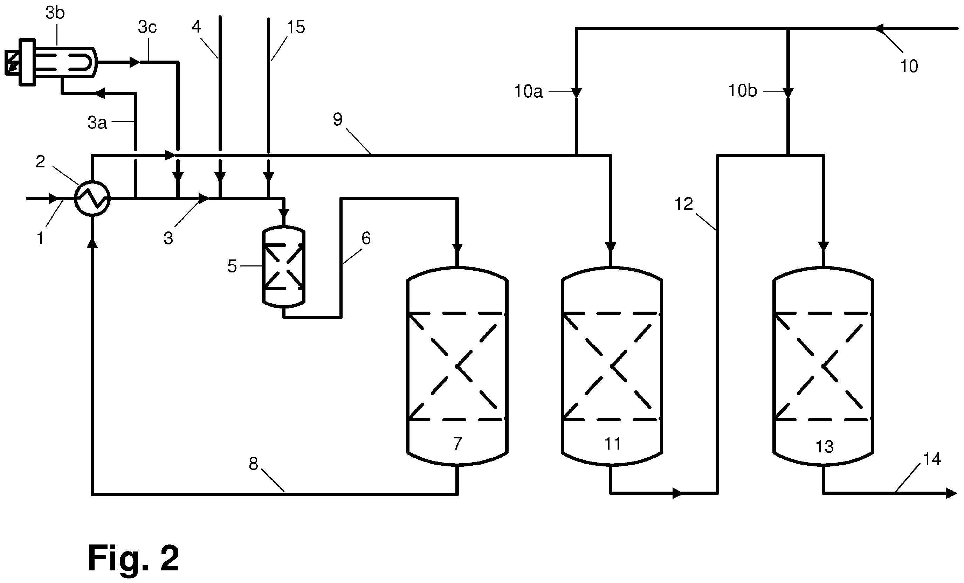

Fig. 2 zeigt eine schematische Darstellung einer beispielhaften Ausgestaltung eines Verfahrens bzw. einer Anlage gemäß der Erfindung. Dabei behalten die Bezugszeichen 1 bis 14 ihre oben erörterte Bedeutung.

Im Unterschied zu der in Fig. 1 gezeigten Ausgestaltung eines Verfahrens bzw. einer Anlage gemäß Stand der Technik wird in Fig. 2 erfindungsgemäß der aus der Gaswaschzone erhaltene, mit Verunreinigungen beladene Methanolspülstrom stromaufwärts des Behälters 5 über Leitung 15 in Leitung 3 zugegeben.

In einer besonderen Ausgestaltung der Erfindung wird der Methanolspülstrom dabei über Leitung 15 in Leitung 3 in flüssiger Form zugegeben. Die Verdampfung erfolgt dann unter Ausnutzung des Wärmeinhalts des in Leitung 3 herangeführten Rohsynthesegases nach Aufheizen in Wärmetauscher 2. Gegebenenfalls kann die Verdampfung dadurch unterstützt werden, dass der in flüssiger Form zugegebene Methanolspülstrom über einen in der Leitung 3 an der Zugabestelle angeordneten Docht, ein Tropfblech oder ähnliche Vorrichtungen zur Vergrößerung der Flüssigkeitsoberfläche geführt wird. Auch eine Zugabe durch Versprühen des Methanolspülstrom mittels einer in Leitung 3 hereinragenden Düse ist möglich.

In einer alternativen, besonderen Ausgestaltung der Erfindung wird der Methanolspülstrom über Leitung 15 in Leitung 3 in Dampfform zugegeben. Die Verdampfung erfolgt dabei vor der Zugabe zu Leitung 3 mittels einer in der Figur nicht gezeigten Verdampfungsvorrichtung, die im Leitungsweg der Leitung 15 angeordnet ist. Vorteilhafterweise ist die Verdampfungsvorrichtung als Wärmetauscher ausgestaltet oder mit einem Wärmetauscher in Fluidverbindung stehend, in dem die Verdampfungsenthalpie dem Methanolspülstrom durch indirekten Wärmetausch mit dem zuvor im Wärmetauscher 2 aufgeheizten Rohsynthesegas erfolgt.

In beiden Ausgestaltungen ist es vorteilhaft, dass die Zugabe des Methanolspülstroms stromaufwärts des Behälter 5, der das Schutzbett enthält, in die CO-Konvertierungszone erfolgt. Vorteilhaft ist es dabei, dass das Schutzbett gleichsam als statischer Mischer genutzt wird, so dass eine homogene Verteilung des Methanolspülstroms in dem Rohsynthesegasproduktstrom erfolgen kann.

### Zahlenbeispiele

In der nachfolgend wiedergegebenen Tabelle werden Stoffmengenströme für wichtige Komponenten miteinander verglichen, die ohne Zugabe (Vergleichsbeispiel) bzw. mit Zugabe (Erfindung) eines Methanolspülstroms zu Leitung 3 stromaufwärts des Behälters 5 ermittelt werden. Angegeben sind jeweils die Stoffmengenströme am Eingang des Behälters 5 und in Leitung 14, also am Ausgang des letzten CO-Konvertierungsreaktors 13.

**Tabelle 1: Stoffmengenströme für wichtige Komponenten ohne bzw. mit Zugabe eines Methanolspülstroms zu Leitung 3 stromaufwärts von Behälter 5**

| | **Ohne MeOH-Spülstrom** | **Mit MeOH-Spülstrom** | **Ohne MeOH-Spülstrom** | **Mit MeOH-Spülstrom** |
|---|---|---|---|---|
| | **Eingang Behälter 5** | **Eingang Behälter 5** | **Leitung 14** | **Leitung 14** |
| | **Vergl.bsp.** | **Erfindung** | **Vergl.bsp.** | **Erfindung** |
| **Stoffmengenstrom kmol/h** | | | | |
| H2 | 2848,23 | 2848,23 | 6021,69 | 6038,62 |
| CO | 3324,91 | 3324,91 | 89,38 | 89,81 |
| CO2 | 352,91 | 352,98 | 3608,72 | 3615,03 |
| H2O | 9008,10 | 9016,65 | 8056,75 | 8057,48 |
| H2S | 44,539 | 44,564 | 46,357 | 46,382 |
| COS | 1,846 | 1,846 | 0,028 | 0,028 |
| NH3 | 16,296 | 16,361 | 18,026 | 18,109 |
| HCN | 1,977 | 1,997 | 0,247 | 0,250 |
| MeOH | 0,240 | 4,871 | 0,351 | 0,357 |

Insbesondere für die Spurenverunreinigung Cyanwasserstoff (HCN) und das Waschmittel Methanol (MeOH) ist eine Reduktion des jeweiligen Stoffmengenstroms nach Durchlaufen der CO-Konvertierungszone deutlich zu erkennen.

### Gewerbliche Anwendbarkeit

Mit der Erfindung wird ein integriertes Verfahren zum Herstellen eines gereinigten und konvertierten Synthesegases bzw. eine entsprechende Anlage bereitgestellt, bei dem der in der Gaswaschzone anfallende Methanolspülstrom und in diesem enthaltene Spurenverunreinigungen zurückgeführt und katalytisch abgebaut werden können. Hierdurch reduzieren sich die Entsorgungskosten und der rückgeführte Anteil des Methanolspülstroms wird stofflich innerhalb des Verfahrens genutzt.

### Bezugszeichenliste

- 1: Leitung
- 2: Wärmetauscher
- 3: Leitung
- 3a: Leitung
- 3b: Inbetriebnahmeheizer
- 3c: Leitung
- 4: Leitung
- 5: Behälter
- 6: Leitung
- 7: Reaktor
- 8: Leitung
- 9: Leitung
- 9a: Leitung
- 9b: Leitung
- 10: Leitung
- 10a: Leitung
- 10b: Leitung
- 11: Reaktor
- 12: Leitung
- 13: Reaktor
- 14: Leitung
- 15: Leitung

## Patentansprüche

1. Verfahren zum Herstellen eines gereinigten und konvertierten Synthesegases, enthaltend Wasserstoff (H₂) und Kohlenmonoxid (CO), umfassend folgende Verfahrensschritte:
a) Bereitstellen eines kohlenstoffhaltigen Vergasungs- oder Reformierungseinsatzstroms und Einleiten in eine Synthesegaserzeugungsstufe,
b) Umsetzen des kohlenstoffhaltigen Vergasungs- oder Reformierungseinsatzstroms in der Synthesegaserzeugungsstufe unter Synthesegaserzeugungsbedingungen zu einem Rohsynthesegasproduktstrom, der neben den Hauptbestandteilen H₂ und CO ferner Kohlendioxid (CO₂) und Schwefelwasserstoff (H₂S) als acide Synthesegasbestandteile sowie Neben- und Spurenkomponenten wie Carbonylsulfid (COS), Ammoniak (NH₃), Cyanwasserstoff (HCN), Mercaptane (RSH), Metallcarbonyle enthält, Ausleiten des Rohsynthesegasproduktstroms aus der Synthesegaserzeugungsstufe,
c) Einleiten des Rohsynthesegasproduktstroms in eine CO-Konvertierungszone, Umsetzen des Rohsynthesegasproduktstroms in der CO-Konvertierungszone unter CO-Konvertierungsbedingungen in einen an H₂ angereicherten und an CO abgereicherten CO-Konvertierungsproduktstrom, Ausleiten des CO-Konvertierungsproduktstroms aus der CO-Konvertierungszone,
d) Einleiten des CO-Konvertierungsproduktstroms in eine nach einem physikalischen Gaswaschverfahren mit Methanol als Absorptionsmittel arbeitenden Gaswaschzone, in der das Methanol im Kreis geführt und kontinuierlich regeneriert wird, Ausleiten eines an aciden Gasbestandteilen wie CO₂ und H₂S abgereicherten Reinsynthesegasproduktstroms aus der Gaswaschzone, Ausleiten eines oder mehrerer an aciden Gasbestandteilen wie CO₂ und H₂S angereicherter Stoffströme aus der Gaswaschzone, Ausleiten eines mit Spurenverunreinigungen beladenen, flüssigen Methanolspülstroms aus der Gaswaschzone,
wobei der Methanolspülstrom mindestens teilweise in die CO-Konvertierungszone zurückgeführt und dem Rohsynthesegasproduktstrom vor Einleiten in die CO-Konvertierungszone zugegeben wird.

2. Verfahren nach Anspruch 1, **dadurch gekennzeichnet, dass** der Rohsynthesegasproduktstrom vor dem Einleiten in die CO-Konvertierungszone ein Schutzbett durchläuft, das mit einem für Metallcarbonyle selektiven Adsorbens oder Absorbens gefüllt ist, wobei der Methanolspülstrom stromaufwärts des Schutzbettes dem Rohsynthesegasproduktstrom zugegeben wird.

3. Verfahren nach Anspruch 2, **dadurch gekennzeichnet, dass** der Methanolspülstrom dem Rohsynthesegasproduktstrom in flüssiger Form zugegeben wird.

4. Verfahren nach Anspruch 2, **dadurch gekennzeichnet, dass** der Methanolspülstrom vor oder während seiner Zugabe zu dem Rohsynthesegasproduktstrom verdampft wird, wobei die Verdampfungswärme durch direkten oder indirekten Wärmetausch mit dem Rohsynthesegasproduktstrom zugeführt wird.

5. Verfahren nach Anspruch 1 bis 4, **dadurch gekennzeichnet, dass** die CO-Konvertierungszone nach dem Prinzip der Rohgaskonvertierung arbeitet.

6. Verfahren nach einem der vorigen Ansprüche, **dadurch gekennzeichnet, dass** die CO-Konvertierungszone mehrere Bereiche umfasst, die mit einem oder mehreren für die Rohgaskonvertierung aktiven Katalysatoren befüllt sind.

7. Verfahren nach einem der vorigen Ansprüche, **dadurch gekennzeichnet, dass** die Gaswaschzone eine Heissregenerierungsvorrichtung zur Regenerierung von mit aciden Gasbestandteilen beladenem Methanol umfasst, wobei der mit Spurenverunreinigungen beladene, flüssigen Methanolspülstrom aus dem Sumpfprodukt und/oder dem Rücklauf der Heissregenerierungsvorrichtung gewonnen und aus der Heissregenerierungsvorrichtung ausgeleitet wird.

8. Anlage zum Herstellen eines gereinigten und konvertierten Synthesegases, enthaltend Wasserstoff (H₂) und Kohlenmonoxid (CO), umfassend folgende, miteinander in Fluidverbindung stehende Bestandteile, Bauelemente und Funktionsgruppen:
a) Eine Synthesegaserzeugungsstufe, Mittel zum Bereitstellen eines kohlenstoffhaltigen Vergasungs- oder Reformierungseinsatzstroms und Mittel zum Einleiten desselben in die Synthesegaserzeugungsstufe,
b) Mittel zum Umsetzen des kohlenstoffhaltigen Vergasungs- oder Reformierungseinsatzstroms in der Synthesegaserzeugungsstufe unter Synthesegaserzeugungsbedingungen zu einem Rohsynthesegasproduktstrom, der neben den Hauptbestandteilen H₂ und CO ferner Kohlendioxid (CO₂) und Schwefelwasserstoff (H₂S) als acide Synthesegasbestandteile sowie Neben- und Spurenkomponenten wie Carbonylsulfid (COS), Ammoniak (NH₃), Cyanwasserstoff (HCN), Mercaptane (RSH), Metallcarbonyle enthält, Mittel zum Ausleiten des Rohsynthesegasproduktstroms aus der Synthesegaserzeugungsstufe,
c) eine CO-Konvertierungszone, Mittel zum Einleiten des Rohsynthesegasproduktstroms in die CO-Konvertierungszone, Mittel zum Umsetzen des Rohsynthesegasproduktstroms in der CO-Konvertierungszone unter CO-Konvertierungsbedingungen in einen an H₂ angereicherten und an CO abgereicherten CO-Konvertierungsproduktstrom, Mittel zum Ausleiten des CO-Konvertierungsproduktstroms aus der CO-Konvertierungszone,
d) eine nach einem physikalischen Gaswaschverfahren mit Methanol als Absorptionsmittel arbeitende Gaswaschzone, in der das Methanol im Kreis geführt und kontinuierlich regeneriert wird, Mittel zum Einleiten des CO-Konvertierungsproduktstroms in die Gaswaschzone, Mittel zum Ausleiten eines an aciden Gasbestandteilen wie CO₂ und H₂S abgereicherten Reinsynthesegasproduktstroms aus der Gaswaschzone, Mittel zum Ausleiten eines oder mehrerer an aciden Gasbestandteilen wie CO₂ und H₂S angereicherter Stoffströme aus der Gaswaschzone, Mittel zum Ausleiten eines mit Spurenverunreinigungen beladenen, flüssigen Methanolspülstroms aus der Gaswaschzone,
wobei ferner Mittel umfasst werden, die es gestatten, den Methanolspülstrom mindestens teilweise in die CO-Konvertierungszone zurückzuführen und dem Rohsynthesegasproduktstrom vor Einleiten in die CO-Konvertierungszone zuzugeben.

9. Anlage nach Anspruch 8, ferner umfassend ein Schutzbett, das mit einem für Metallcarbonyle selektiven Adsorbens oder Absorbens gefüllt ist und das stromaufwärts der CO-Konvertierungszone angeordnet ist, so dass der Rohsynthesegasproduktstrom vor dem Einleiten in die CO-Konvertierungszone das Schutzbett durchläuft, und ferner umfassend Mittel, die es gestatten, dass der Methanolspülstrom stromaufwärts des Schutzbettes dem Rohsynthesegasproduktstrom zugegeben wird.

10. Anlage nach Anspruch 8 oder 9, ferner umfassend Mittel, die es gestatten, dass der Methanolspülstrom dem Rohsynthesegasproduktstrom in flüssiger Form zugegeben wird.

11. Anlage nach Anspruch 8 oder 9, ferner umfassend Mittel, die es gestatten, dass der Methanolspülstrom vor oder während seiner Zugabe zu dem Rohsynthesegasproduktstrom verdampft wird, wobei die Verdampfungswärme durch direkten oder indirekten Wärmetausch mit dem Rohsynthesegasproduktstrom zugeführt wird.

12. Anlage nach Anspruch 8 bis 11, **dadurch gekennzeichnet, dass** die CO-Konvertierungszone nach dem Prinzip der Rohgaskonvertierung arbeitet.

13. Anlage nach einem der vorigen Ansprüche, **dadurch gekennzeichnet, dass** die CO-Konvertierungszone mehrere Bereiche umfasst, die mit einem oder mehreren für die Rohgaskonvertierung aktiven Katalysatoren befüllt sind.

14. Anlage nach einem der vorigen Ansprüche, **dadurch gekennzeichnet, dass** die Gaswaschzone eine Heissregenerierungsvorrichtung zur Regenerierung von mit aciden Gasbestandteilen beladenem Methanol umfasst, wobei ferner Mittel umfasst werden, die es gestatten, dass der mit Spurenverunreinigungen beladene, flüssige Methanolspülstrom aus dem Sumpfprodukt und/oder dem Rücklauf der Heissregenerierungsvorrichtung gewonnen und aus der Heissregenerierungsvorrichtung ausgeleitet wird.

## Claims

1. Process for producing a purified and converted synthesis gas containing hydrogen (H₂) and carbon monoxide (CO), comprising the following process steps:
a) providing a carbon-containing gasification or reforming input stream and introducing said stream into a synthesis gas generation stage,
b) reacting the carbon-containing gasification or reforming input stream in the synthesis gas generation stage under synthesis gas generation conditions to afford a raw synthesis gas product stream which contains not only the primary constituents H₂ and CO but also carbon dioxide (CO₂) and hydrogen sulfide (H₂S) as acidic synthesis gas constituents as well as secondary and trace components such as carbonyl sulfide (COS), ammonia (NH₃), hydrogen cyanide (HCN), mercaptans (RSH), metal carbonyls, discharging the raw synthesis gas product stream from the synthesis gas generation stage,
c) introducing the raw synthesis gas product stream into a CO conversion zone, converting the raw synthesis gas product stream in the CO conversion zone under CO conversion conditions into an H₂ -enriched and CO-depleted CO conversion product stream, discharging the CO conversion product stream from the CO conversion zone,
d) introducing the CO conversion product stream into a gas scrubbing zone operating according to a physical gas scrubbing process with methanol as the absorption medium in which the methanol is recirculated and is continuously regenerated, discharging a pure synthesis gas product stream depleted of acidic gas constituents such as CO₂ and H₂S from the gas scrubbing zone, discharging one or more material streams enriched in acidic gas constituents such as CO₂ and H₂S from the gas scrubbing zone, discharging a liquid methanol purge stream laden with trace impurities from the gas scrubbing zone,
wherein the methanol purge stream is at least partially recycled into the CO conversion zone and added to the raw synthesis gas product stream before introduction into the CO conversion zone.

2. Process according to Claim 1, **characterized in that** before introduction into the CO conversion zone the raw synthesis gas product stream passes through a protective bed filled with an adsorbent or absorbent selective for metal carbonyls, wherein the methanol purge stream is added to the raw synthesis gas product stream upstream of the protective bed.

3. Process according to Claim 2, **characterized in that** the methanol purge stream is added to the raw synthesis gas product stream in liquid form.

4. Process according to Claim 2, **characterized in that** the methanol purge stream is evaporated before or during its addition to the raw synthesis gas product stream, wherein the evaporation heat is supplied by direct or indirect heat exchange with the raw synthesis gas product stream.

5. Process according to any of Claims 1 to 4, **characterized in that** the CO conversion zone operates according to the principle of raw gas conversion.

6. Process according to any of the preceding claims, **characterized in that** the CO conversion zone comprises a plurality of regions filled with one or more catalysts active for the raw gas conversion.

7. Process according to any of the preceding claims, **characterized in that** the gas scrubbing zone comprises a hot regeneration apparatus for regeneration of methanol laden with acidic gas constituents, wherein the liquid methanol purge stream laden with trace impurities is obtained from the bottoms product and/or the reflux from the hot regeneration apparatus and is discharged from the hot regeneration apparatus.

8. Plant for producing a purified and converted synthesis gas containing hydrogen (H₂) and carbon monoxide (CO), comprising the following constituents, constructional elements and functional groupings in fluid connection with one another:
a) a synthesis gas generation stage, means for providing a carbon-containing gasification or reforming input stream and means for introducing said stream into the synthesis gas generation stage,
b) means for reacting the carbon-containing gasification or reforming input stream in the synthesis gas generation stage under synthesis gas generation conditions to afford a raw synthesis gas product stream which contains not only the primary constituents H₂ and CO but also carbon dioxide (CO₂) and hydrogen sulfide (H₂S) as acidic synthesis gas constituents as well as secondary and trace components such as carbonyl sulfide (COS), ammonia (NH₃), hydrogen cyanide (HCN), mercaptans (RSH), metal carbonyls, means for discharging the raw synthesis gas product stream from the synthesis gas generation stage,
c) a CO conversion zone, means for introducing the raw synthesis gas product stream into the CO conversion zone, means for converting the raw synthesis gas product stream in the CO conversion zone under CO conversion conditions into an H₂-enriched and CO-depleted CO conversion product stream, means for discharging the CO conversion product stream from the CO conversion zone,
d) a gas scrubbing zone operating according to a physical gas scrubbing process with methanol as the absorption medium in which the methanol is recirculated and continuously regenerated, means for introducing the CO conversion product stream into the gas scrubbing zone, means for discharging a pure synthesis gas product stream depleted of acidic gas constituents such as CO₂ and H₂S from the gas scrubbing zone, means for discharging one or more material streams enriched in acidic gas constituents such as CO₂ and H₂S from the gas scrubbing zone, means for discharging a liquid methanol purge stream laden with trace impurities from the gas scrubbing zone,
wherein said plant further comprises means which make it possible for the methanol purge stream to be at least partially recycled into the CO conversion zone and added to the raw synthesis gas product stream before introduction into the CO conversion zone.

9. Plant according to Claim 8, further comprising a protective bed filled with an adsorbent or absorbent selective for metal carbonyls and arranged upstream of the CO conversion zone so that the raw synthesis gas product stream passes through the protective bed before introduction into the CO conversion zone and further comprising means allowing the methanol purge stream to be added to the raw synthesis gas product stream upstream of the protective bed.

10. Plant according to Claim 8 or 9, further comprising means allowing the methanol purge stream to be added to the raw synthesis gas product stream in liquid form.

11. Plant according to Claim 8 or 9, further comprising means allowing the methanol purge stream to be evaporated before or during its addition to the raw synthesis gas product stream, wherein the evaporation heat is supplied by direct or indirect heat exchange with the raw synthesis gas product stream.

12. Plant according to any of Claims 8 to 11, **characterized in that** the CO conversion zone operates according to the principle of raw gas conversion.

13. Plant according to any of the preceding claims, **characterized in that** the CO conversion zone comprises a plurality of regions filled with one or more catalysts active for the raw gas conversion.

14. Plant according to any of the preceding claims, **characterized in that** the gas scrubbing zone comprises a hot regeneration apparatus for regeneration of methanol laden with acidic gas constituents, wherein said plant further comprises means allowing the liquid methanol purge stream laden with trace impurities to be obtained from the bottoms product and/or the reflux from the hot regeneration apparatus and discharged from the hot regeneration apparatus.

## Revendications

1. Procédé pour la production d'un gaz de synthèse épuré et converti, contenant de l'hydrogène (H₂) et du monoxyde de carbone (CO), comprenant les étapes de processus suivantes :
a) fourniture d'un courant d'alimentation de reformage ou gazéification contenant du carbone et introduction dans un étage de production de gaz de synthèse,
b) transformation du courant d'alimentation de reformage ou gazéification contenant du carbone dans l'étage de production de gaz de synthèse, dans des conditions de production de gaz de synthèse, en un courant de produit de gaz de synthèse brut qui, en plus des constituants principaux H₂ et CO, contient en outre du dioxyde de carbone (CO₂) et du sulfure d'hydrogène (H₂S) en tant que constituants acides de gaz de synthèse ainsi que des composants accessoires et à l'état de traces tels que du sulfure de carbonyle (COS), de l'ammoniac (NH₃) , de l'acide cyanhydrique (HCN), des mercaptans (RSH), des carbonyles de métaux, évacuation du courant de produit de gaz de synthèse brut hors de l'étage de production de gaz de synthèse,
c) introduction du courant de produit de gaz de synthèse brut dans une zone de conversion de CO, transformation du courant de produit de gaz de synthèse brut dans la zone de conversion de CO, dans des conditions de conversion de CO, en un courant de produit de conversion de CO enrichi en H₂ et appauvri en CO, évacuation du courant de produit de conversion de CO hors de la zone de conversion de CO,
d) introduction du courant de produit de conversion de CO dans une zone de lavage de gaz fonctionnant selon un procédé de lavage physique de gaz avec du méthanol en tant qu'agent d'absorption, dans laquelle le méthanol est mis en circuit et régénéré en continu, évacuation hors de la zone de lavage de gaz d'un courant de produit de gaz de synthèse pur appauvri en constituants acides de gaz tels que CO₂ et H₂S, évacuation hors de la zone de lavage de gaz d'un ou de plusieurs courants de substances enrichis en constituants acides de gaz tels que CO₂ et H₂S, évacuation hors de la zone de lavage de gaz d'un courant liquide de rinçage de méthanol chargé d'impuretés à l'état de traces,
le courant de rinçage de méthanol étant au moins partiellement renvoyé dans la zone de conversion de CO et ajouté au courant de produit de gaz de synthèse brut avant introduction dans la zone de conversion de CO.

2. Procédé selon la revendication 1, **caractérisé en ce qu'**avant l'introduction dans la zone de conversion de CO le courant de produit de gaz de synthèse brut passe à travers un lit protecteur qui est rempli avec un absorbant ou adsorbant sélectif pour les carbonyles de métaux, le courant de rinçage de méthanol étant ajouté au courant de produit de gaz de synthèse brut en amont du lit protecteur.

3. Procédé selon la revendication 2, **caractérisé en ce que** le courant de rinçage de méthanol est ajouté sous forme liquide au courant de produit de gaz de synthèse brut.

4. Procédé selon la revendication 2, **caractérisé en ce que** le courant de rinçage de méthanol est vaporisé avant ou pendant son addition au courant de produit de gaz de synthèse brut, la chaleur de vaporisation étant amenée par échange direct ou indirect de chaleur avec le courant de produit de gaz de synthèse brut.

5. Procédé selon l'une des revendications 1 à 4, **caractérisé en ce que** la zone de conversion de CO fonctionne selon le principe de la conversion de gaz brut.

6. Procédé selon l'une des revendications précédentes, **caractérisé en ce que** la zone de conversion de CO comprend plusieurs secteurs qui sont remplis avec un ou plusieurs catalyseurs actifs pour la conversion de gaz brut.

7. Procédé selon l'une des revendications précédentes, **caractérisé en ce que** la zone de lavage de gaz comprend un dispositif de régénération à chaud destiné à la régénération de méthanol chargé de constituants acides de gaz, le courant liquide de rinçage de méthanol, chargé d'impuretés à l'état de traces, étant obtenu à partir du produit de pied et/ou du reflux du dispositif de régénération à chaud et évacué hors du dispositif de régénération à chaud.

8. Unité destinée à la production d'un gaz de synthèse épuré et converti, contenant de l'hydrogène (H₂) et du monoxyde de carbone (CO), comprenant les constituants, composants et groupes fonctionnels suivants, en communication fluidique entre eux :
a) un étage de production de gaz de synthèse, des moyens destinés à la fourniture d'un courant d'alimentation de reformage ou gazéification contenant du carbone et des moyens destinés à l'introduction de celui-ci dans l'étage de production de gaz de synthèse,
b) des moyens destinés à la transformation du courant d'alimentation de reformage ou gazéification contenant du carbone dans l'étage de production de gaz de synthèse, dans des conditions de production de gaz de synthèse, en un courant de produit de gaz de synthèse brut qui, en plus des constituants principaux H₂ et CO, contient en outre du dioxyde de carbone (CO₂) et du sulfure d'hydrogène (H₂S) en tant que constituants acides de gaz de synthèse ainsi que des composants accessoires et à l'état de traces tels que du sulfure de carbonyle (COS), de l'ammoniac (NH₃), de l'acide cyanhydrique (HCN), des mercaptans (RSH), des carbonyles de métaux, des moyens destinés à l'évacuation du courant de produit de gaz de synthèse brut hors de l'étage de production de gaz de synthèse,
c) une zone de conversion de CO, des moyens destinés à l'introduction du courant de produit de gaz de synthèse brut dans la zone de conversion de CO, des moyens destinés à la transformation du courant de produit de gaz de synthèse brut dans la zone de conversion de CO, dans des conditions de conversion de CO, en un courant de produit de conversion de CO enrichi en H₂ et appauvri en CO, des moyens destinés à l'évacuation du courant de produit de conversion de CO hors de la zone de conversion de CO,
d) une zone de lavage de gaz fonctionnant selon un procédé de lavage physique de gaz avec du méthanol en tant qu'agent d'absorption, dans laquelle le méthanol est mis en circuit et régénéré en continu, des moyens destinés à l'introduction du courant de produit de conversion de CO dans la zone de lavage de gaz, des moyens destinés à l'évacuation hors de la zone de lavage de gaz d'un courant de produit de gaz de synthèse pur appauvri en constituants acides de gaz tels que CO₂ et H₂S, des moyens destinés à l'évacuation hors de la zone de lavage de gaz d'un ou de plusieurs courants de substances enrichis en constituants acides de gaz tels que CO₂ et H₂S, des moyens destinés à l'évacuation hors de la zone de lavage de gaz d'un courant liquide de rinçage de méthanol chargé d'impuretés à l'état de traces,
étant en outre compris des moyens qui permettent de renvoyer le courant de rinçage de méthanol au moins partiellement dans la zone de conversion de CO et de l'ajouter au courant de produit de gaz de synthèse brut avant introduction dans la zone de conversion de CO.

9. Unité selon la revendication 8, comprenant en outre un lit protecteur qui est rempli avec un absorbant ou adsorbant sélectif pour les carbonyles de métaux et qui est disposé en amont de la zone de conversion de CO, de sorte qu'avant l'introduction dans la zone de conversion de CO le courant de produit de gaz de synthèse brut passe à travers le lit protecteur, et comprenant en outre des moyens qui permettent d'ajouter le courant de rinçage de méthanol au courant de produit de gaz de synthèse brut en amont du lit protecteur.

10. Unité selon la revendication 8 ou 9, comprenant en outre des moyens qui permettent d'ajouter sous forme liquide le courant de rinçage de méthanol au courant de produit de gaz de synthèse brut.

11. Unité selon la revendication 8 ou 9, comprenant en outre des moyens qui permettent de vaporiser le courant de rinçage de méthanol avant ou pendant son addition au courant de produit de gaz de synthèse brut, la chaleur de vaporisation étant amenée par échange direct ou indirect de chaleur avec le courant de produit de gaz de synthèse brut.

12. Unité selon l'une des revendications 8 à 11, **caractérisée en ce que** la zone de conversion de CO fonctionne selon le principe de la conversion de gaz brut.

13. Unité selon l'une des revendications précédentes, **caractérisée en ce que** la zone de conversion de CO comprend plusieurs secteurs qui sont remplis avec un ou plusieurs catalyseurs actifs pour la conversion de gaz brut.

14. Unité selon l'une des revendications précédentes, **caractérisée en ce que** la zone de lavage de gaz comprend un dispositif de régénération à chaud destiné à la régénération de méthanol chargé de constituants acides de gaz, étant en outre compris des moyens qui permettent d'obtenir le courant liquide de rinçage de méthanol, chargé d'impuretés à l'état de traces, à partir du produit de pied et/ou du reflux du dispositif de régénération à chaud et de l'évacuer hors du dispositif de régénération à chaud.
